# EUROPÄISCHE PATENTSCHRIFT

(11) **EP 1 029 545 B1**
(45) Veröffentlichungstag und Bekanntmachung des Hinweises auf die Patenterteilung: **09.11.2005**
(21) Anmeldenummer: 00103176.4
(22) Anmeldetag: 16.02.2000
(51) Int. Cl.: A61K 35/80, A61P 19/00, A61P 3/00

(54) **Zubereitung für oral zu verabreichende Nahrungsergänzungs- und/oder Arznelmittel in Form von Tabletten**
Composition for oral administration of nutritional supplement or medicaments in tablet form
Composition orale pour l'administration de compléments alimentaires ou de médicaments sous forme de tablettes

(30) Priorität: 16.02.1999 DE 19906016
(43) Veröffentlichungstag der Anmeldung: 23.08.2000
(73) Patentinhaber: GreenPower International Natuurprodukten B.V., 6433 KC Heerlen-Hoensbroeck (NL)
(72) Erfinder: HITTICH, Reinhard Dr., NL-6466 NHKerkrade (NL)
(74) Vertreter: Cronin, Brian Harold John

(56) Entgegenhaltungen:
- EP-A- 0 504 508
- DE-A- 19 608 563
- US-A- 4 006 254

## Beschreibung

Die Erfindung betrifft eine Zubereitung gemäß dem Ansprüchen für oral zu verabreichende Nahrungsergänzungsund/oder Arzneimittel in Form von Tabletten für Mensch und/oder Tier auf der Basis von pflanzlichen Material natürlichen Ursprungs und unter Verwendung von kalkbildenden Algen und/oder kieselsäurebildenden Algen und/oder Teilen dieser Algen.

Nahrungsergänzungsstoffe dieser Art sind im Stand der Technik bekannt. So beschreibt z.B. das Schutzrecht DE 34 21 644 C2 einen Nahrungs- bzw. Futterzusatz, der als mengenmäßige Hauptkomponente einen Trockenextrakt der Blaualge Spirulina enthält. Der Spirulina-Trocken-extrakt dient sowohl als Wirkstoffquelle als auch als Trägermaterial für zusätzliche Wirkstoffe.

Aus der DE 196 08 563 A1 sind Zubereitungen für Nahrungsergänzungs- und/oder Arzneimittel bekannt, die unter Verwendung von kalkbildenden und/oder kieselsäurebildenden Algen bzw. Algenteilen und von chlorophyllreichem, pflanzlichem Material hergestellt sind.

Diese Zubereitungen sind an sich bereits sehr vorteilhaft: Sie bestehen vollständig oder zumindest überwiegend aus pflanzlichen Naturstoffen, die üblicherweise in ihrer natürlichen Umgebung und unter natürlichen Bedingungen entstanden bzw. gewachsen sind und in weitgehend naturbelassener Form eingesetzt werden, weshalb sie in aller Regel keine Stoffe enthalten, die für den menschlichen oder tierischen Körper ein Allergen darstellen und dementsprechend allergische Reaktionen hervorrufen.

Das Algenmaterial läßt sich technisch einfach zu Tabletten verpressen. Seine Mischbarkeit mit sehr vielen Wirkstoffen ist ausgesprochen gut, die Wirkstoffe können ungehindert vom Körper resorbiert werden, und das Algenmaterial selbst enthält zahlreiche Mikronährstoffe und Spurenelemente in natürlicher Form und Verteilung, die für sämtliche enzymatischen Vorgänge im menschlichen Körper von grundsätzlicher Bedeutung sind und ihm somit ebenfalls zur Verfügung gestellt werden. Im Gegensatz zu vielen herkömmlichen Supplementierungen einzelner Spurenelemente in hohen Einzeldosen enthält das beschriebene Algenmaterial alle Spurenelemente in natürlicher Form, Konzentration und Zusammensetzung, so daß sich der Körper bzw. die betreffenden Körperzellen holen können, was sie benötigen. Die kalkbildenden Algen sind reich an dem lebensnotwendigen Mineral Calcium, das insbesondere für Knochenaufbau, Zahnbildung, Muskel- und Nervenfunktionen und den Herzkreislauf von elementarer Bedeutung ist. Es wird allerdings häufig nur in ungenügenden Mengen mit der täglichen Nahrung aufgenommen. Die kieselsäurebildenden Algen mit ihrem hohen Kieselsäuregehalt stärken die körpereigenen Abwehrkräfte und fördern den Stoffwechsel.

Das chlorophyllreiche Material hat den Vorteil eines besonders hohen Gehalts an gespeicherter Sonnenlichtenergie. Pflanzen können gewaltige Rationen von Sonnenphotonen speichern. Nicht der Gehalt an Kalorien und Mikronährstoffen allein ist es, was den Wert der Lebensmittel ausmacht. Der Informationsgehalt in Form von gespeicherten Sonnenphotonen, die Biophotonen-Aktivitat, bestimmt in hohem Maße den wirklichen Wert unserer Nahrung. Die Biophotonenstrahlung wurde bereits im Jahre 1922 von dem russischen Mediziner Prof. Alexander Gurwitsch erstmals festgestellt. Seit 1975 beweisen deutsche Biophysiker unter der Leitung von Professor Fritz-Albert Popp mit modernsten Forschungsmethoden ständig mehr, wie lebenswichtig dieses Qualitätskriterium "lebendiger" Nahrung für die menschliche bzw. tierische Gesundheit ist. (Teubner, R.; Rattemeyer, M. und Mehlhardt, W.: Eine neue Methode zur Untersuchung der Qualität von Pflanzen und Früchten, Ärztezeitschrift f. Naturheilverfahren, 4, 204-205 (1981).― Popp, F.A.: Biophotonen-Analyse der Lebensmittelqualität. In: Lebensmittelqualität - Ganzheitliche Methoden und Konzepte. C. F. Müller, Karlsruhe (1988) p. 87-112. ― Köhler, B.; Lambing, K.; Neurohr, W.; Nagl, W.; Popp, F.A. und Wahler, J.: Photonenemission - Eine neue Methode zur Erfassung der "Qualität" von Lebensmitteln. Deutsche Lebensmittelrundschau, 3, 78-83 (1991).― Lambing, K.: Biophoton measurement as a supplement to the conventional consideration of food quality. In: Recent advances in biophoton research and its application. World Scientific, Singapore-New Jersey-London-Hong Kong (1992) p. 393-413. ― Lambing K.: Nutzung der "low level luminescence" Meßtechnik zur Untersuchung von Lebensmitteln, Dissertationsschrift, angefertigt an der Universität Kaiserslautern (1992).

― Popp, F.A.: Die Botschaft der Nahrung. Fischer Taschenbuch, Frankfurt (1993). - BGVV/Bundesamt für gesundheitlichen Verbraucherschutz und Veterinärmedizin, K.-H. Engel, G.A. Schreiber, K.W. Bögl (Hrsg.); Entwicklung von Methoden zum Nachweis mit Hilfe gentechnischer Verfahren hergestellter Lebensmittel - Ein Statusbericht, 01/1995. - Marco Bischof "Biophotonen - das Licht in unseren Zellen", Verlag 2001, Frankfurt 1995, ISBN 3-86150-095-7 und dort zitierte Literatur.

Zur Herstellung von qualitativ hochwertigen Tabletten aus chlorophyllreichem Material, d. h. von Tabletten, die den gleichen Ernährungswert haben, wie das chlorophyllreiche, pulverförnüge Ausgangsmaterial sind im Stand der Technik zwei alternative Vorgehensweisen bekannt:

Zum einen ist aus dem Interview 'The Power and Benefit of Spirulina" (http:// www. sedonanaturals.com/transcript.html, 25.01.1999) bekannt, daß bei der Tablettierung von pulverisierten Spirulinaalgen auf künstliche Füll- und Hilfsstoffe wie Binde-, Fließ- und Trennmittel vollständig verzichtet werden kann. Zur Tablettierung wird ausschließlich reines Spirulinapulver eingesetzt, und das Tablettierverfahren ist dadurch charakterisiert, daß die Tabletten mittels Pressdruck geformt werden, und daß die Tablettiervorrichtung langsamer betrieben und häufiger gereinigt wird, als dies herkömmlicherweise erfolgt. Zum anderen ist aus der Produktbeschreibung "Sonnenkraft im Zauberglas, Spirulina von Marcus Rohrer" (Spirulina International bv, Postfach 800, NL-5000 AV Tilburg) bekannt, daß die Qualität von chlorophyllreichem Material als Nahrungsergänzungs- oder Arzneistoff mit dem Gehalt an gespeicherter Sonnenenergie, d.h. an gespeicherten Sonnenphotonen (die "Biophotonen-Aktivität") korreliert, und daß die von Natur aus hohe ganzheitliche Qualität von Spirulinaalgen, d.h. ihr Gehalt an Kalorien, Mikronährstoffen und gespeicherten Sonnenphotonen,. nach der Pulversierung und Tablettierung dadurch erhalten werden kann, daß Pulver bzw. Tabletten in Violettglasverpackung aufbewahrt werden.

Der vorliegenden Erfindung liegt nun die Aufgabe zugrunde, die aus dem Stand der Technik bekannten Tabletten aus Biophotonen speicherndem pflanzlichen Material weiter zu optimieren, insbesondere deren ganzheitliche Qualität weiter zu verbessern, d. h. Qualitätsverluste im Vergleich zum pulverisierten Ausgangsmaterial (Rohmaterial) sollen vermieden werden, und zwar sowohl zum Zeitpunkt des Tablettierens als auch während der Lagerung im Packmittel.

Eine Lösung dieser Aufgabe besteht in der Bereitstellung einer Zubereitung der eingangs genannten Art, die sich dadurch auszeichnet, daß sie ein natürliches Antioxidans gemäß dem Ansprüchen als Zusatz (Additiv) enthält. Unter einem natürlichen Antioxidans wird in diesem Zusammenhang ein Naturstoff mit antioxidativer Wirkung oder ein naturidentischer synthetischer Stoff mit antioxidativer Wirkung verstanden.

Diese erfindungsgemäße Zubereitung hat gegenüber den bekannten Zubereitungen den überraschenden und allen Erwartungen zuwider laufenden Vorteil, daß daraus hergestelle Tabletten eine höhere ganzheitliche Qualität aufweisen als Tabletten, die ausschließlich, d.h. zu 100% aus dem jeweils betreffenden pflanzlichen Material bestehen. Die kombinierte Verwendung von kalkbildenden und/oder kieselsäurebildenden Algen bzw. Teilen dieser Algen und eines natürlichen Antioxidans bei der Tablettierung des pflanzlichen Materials ergab überraschend die bei weitem beste ganzheitliche Qualität. Offensichtlich bewirkt die Kombination aus gewünschtem pflanzlichen Material und einem Zusatz an natürlichem Antioxidans eine Stabilisierung oder gar Steigerung der ursprünglichen ganzheitlichen Qualität des pflanzlichen Materials, so daß Verluste dieser Qualität während des Tablettierungsvorgangs entweder verringert, vermieden oder kompensiert werden.

In einer Variante der Erfindung besteht das pflanzliche Material vorzugsweise aus chlorophyllreichem, pflanzlichen Material und insbesondere ganz oder teilweise aus Blaualgen (Cyanophyceae) und/oder Grünalgen (Chlorophyceae) und/oder Braunalgen (Phaeophyceae) und/oder Teilen davon. Bevorzugt ist eine Ausführungsform, bei der die Blaualgen aus der systematischen Gruppe Spirulina ausgewählt sind. Spirulina ist eine mikroskopisch kleine Frischwasseralge, die kultiviert und relativ schnell in großem Umfang vermehrt werden kann. Ihre Zellen weisen einen besonders hohen Gehalt an Vitamin B-12, Vitamin E, Beta-Carotin, organischem Eisen, und Gamma-Linolensäure auf und können vom menschlichen bzw. tierischen Organismus gut aufgeschlossen bzw. verdaut werden.

Das chlorophyllreiche Material kann aber ebensogut ganz oder teilweise aus chlorophyllreichem Extrakt höherer Pflanzen bestehen. Bei den höheren Pflanzen handelt es sich vorzugsweise um Gräser, insbesondere um Triticum aestivum (Weizen) und/oder Hordeum vulgare (Gerste) und/oder Kamut, (eine unter dem Namen QK-77 beim amerikanischen Landwirtschaftsministerium anerkannte Getreidesorte mit einem besonders günstigen Nährstoffprofil), oder auch um Medicago sativa (Luzerne; Alfalfa).

Das pflanzliche Material kann aber ebensogut ganz oder teilweise aus Früchten höherer Pflanzen bestehen, bzw. aus dem schonend getrockneten Pulver aus Fruchtsäften und/oder ganzen Früchten. Bei den höheren Pflanzen handelt es sich vorzugsweise um zur menschlichen Ernährung geeigneten Früchten oder Gemüsen.

Als kalkbildende Algen sind Rotalgen (Rhodophyceae) besonders bevorzugt, und unter diesen vor allem die systematische Gruppe Lithothamnium, insbesondere die Art Lithothamnium calcareum. Lithothamnium calcareum wächst in sauerstoffreichem Wasser in Tiefen zwischen 20 und 50 m und erreicht einen Durchmesser von ca. 30 cm. Frisches Algenmaterial, d.h. lebende Vegetationskörper oder dessen Teile können auf einfachste Art und Weise dadurch erhalten werden, daß durch Sturm und Gezeitenwechsel abgerissene Vegetationskörper/-teile, die sich in den zahlreich vorhandenen natürlichen Senken und Graben am Meeresboden angesammelt haben, daraus abgesaugt werden. Das fossile Material wird mechanisch am Meeresboden gewonnen.

Als kieselsäurebildende Algen werden vorzugsweise Kieselalgen (Bacillariophyceae; Diatomeae) eingesetzt. Bei Verwendung von kieselsäurebildenden Algen bzw. Algenteilen ist der Einsatz von Kieselgur besonders bevorzugt.

In der erfindungsgemäßen Zubereitung beträgt der Gehalt an pflanzlichen Material vorzugsweise wenigstens 40 Gewichtsprozent, insbesondere wenigstens 60 Gewichtsprozent und besonders bevorzugt wenigstens 70 Gewichtsprozent.

Für den Fall, daß die Zubereitung bzw. die daraus hergestellten Tabletten ausschließlich aus pflanzlichem Material, Kalk- oder Kieselalgen und Antioxidans bestehen kann oder soll, wird vorgeschlagen, daß der Gehalt an pflanzlichem Material wenigstens 70 Gewichtsprozent, vorzugsweise wenigstens 80 Gewichtsprozent und besonders bevorzugt wenigstens 90 Gewichtsprozent beträgt.

Der Gehalt an kalkbildenden und/oder kieselsäurebildenden Algen bzw. Algenteilen sollte ― bezogen auf das Gesamtgewicht der Zubereitung, ― 0,1 bis 50 Gewichts-%, vorzugsweise von 0,3 bis 35 % und insbesondere von 10 bis 30 Gewichts-% betragen, wenn eine Zubereitung bzw. daraus hergestellte Tabletten mit hohem Anteil an Mineral- und Spurenelementen gewünscht sind. Für den Fall, daß auf die Mineral- und Spurenelemente weitgehend verzichtet werden kann oder gar soll, sieht die Erfindung eine Zubereitungsvariante vor, bei der der Gehalt an kalkbildenden Algen und/oder kieselsäurebildenden Algen und/oder Teilen dieser Algen vorzugsweise nur 0,05 bis 10 Gewichts-%, vorzugsweise 0,1 bis 3 % und insbesondere 0,3 bis 1 Gewichts-%, jeweils bezogen auf das Gesamtgewicht der Zubereitung, beträgt.

Als Antioxidans sind im Prinzip alle bekannten natürlichen oder naturidentischen Stoffe mit antioxidativer Wirkung geeignet - und ganz besonders alle in Europa bzw. Deutschland als Zusatzstoffe mit antioxidativer Wirkung zugelassenen Zusatzstoffe gemäß Verordnung über die Zulassung von Zusatzstoffen zu Lebensmitteln. Der Einsatz von natürlichen bzw. naturiden-tischen Antioxidantien hat den Vorteil, daß damit keine der für den tierischen und insbesondere menschlichen Organismus negative Eigenschaften einhergehen, die für die herkömmlicherweise in diätischen Nahrungsmitteln u.a eingesetzten Antioxidantien bekannt sind. Zu den oben genannten zugelassenen Zusatzstoffen mit antioxidativer Wirkung gehören insbesondere *Astragalus sp*. (Leguminosae/Fabaceae), *Piper nigrum* (Piperaceae), *Capsicum sp*. (Solanaceae), *Apium graveolens* (Umbellifereae), *Cacao theobroma* (Sterculiaceae), *Eleutherococcus senticosus* (Araliaceae), *Zingiber officinalis* (Zingiberaceae), *Pueraria lobata* (Leguminosea/Fabaceae), *Citrus limon* oder *Citrus paradisi* (Rufaceae), *Vitis vinifera* (Vitaceae), *Sylibum marianum*, (Asteraceae/Compositae), *Boswellia sp*. (Burseraceae), *Rosmarinus officinalis*, (Labiatea/Laminaceae), *Salvia officinalis* (Labiatae/Laminaceae), *Schisandra sp*. (Schisandraceae) und *Camellia sinensis* (Theaceac).

In der Praxis haben sich insbesondere die Curcumin-reichen Trockenextrakte von Curcuma sp. (Zingiberaceae) und/oder wässrige Extrakte von Curcuma sp. (sog. Turmeric-Extrakte mit hohem Proteingehalt)) und/oder Vitamin **C.**

Der Einsatz von Curcuma-Extrakten hat den Vorteil, daß die Zubereitung damit zusätzliche, therapeutisch wirksame Inhaltsstoffe aufweist: Curcuma-Trockenextrakt bzw. dessen Hauptinhaltsstoff Curcumin ist in der Naturheilkunde bekannt für seine entzündungshemmende, antiarthritische, antioxidative, antiallergische, antibakterielle und anti-Tumor-Aktivität.

Für den wässerigen Curcuma-Extrakt (Turmeric-Extrakt) ist in der Naturheilkunde-Literatur eine magenberuhigende, blutreinigende und wundheilungsfördernde Wirkung beschrieben.

Und Vitamin **C** ist nachgewiesenermaßen ein lebensnotwendiger Stoff, den der menschliche Körper in relativ großen Mengen benötigt, aber nicht selbst synthetisieren kann und deshalb mit der Nahrung aufnehmen muß. Vitamin C-Mangel kann zu schweren Krankheiten führen.

Die erfindungsgemäßen Zubereitungen bzw. Tabletten können als weiteren Zusatzstoff (Additiv) ein Sprengmittel enthalten. Das Sprengmittel stammt vorzugsweise aus der Gruppe der natürlichen Nahrungs- oder Nahrungsergänzungsstoffe, so daß auch diese Zubereitungen bzw. Tabletten ausschließlich aus Naturstoffen besteht, und überdies aus solchen, die vom menschlichen bzw. tierischen Organismus auch natürlicherweise aufgenommen werden oder zumindest aufgenommen werden können. In der Praxis haben sich als Sprengmittel insbesondere Reis und/oder Kartoffelmehl gut bewährt.

Es besteht ferner die Möglichkeit, die erfindungsgemäße Zubereitung mit geschmacksgebenden Zusätzen zu versetzen, wobei es sich vorzugsweise um Naturstoffe oder naturidentische Stoffe, wie beispielsweise Aromastoffe oder ätherische Öle, und insbesondere um Kakao oder Carob handelt. Es können auch Pulver von Pflanzen, die zur Gewinnung von ätherischen Ölen dienen, eingesetzt werden. Nicht zuletzt können sämtliche erfindungsgemäßen bzw. erfindungsgemäß hergestellten Tabletten mit einem beliebigen bekannten Dragierüberzug versehen sein.

Zur weiteren Veranschaulichung der Erfindung sind nachfolgend Rezepturen von bevorzugten erfindungsgemäßen Zubereitungen und von Vergleichsbeispielen aus dem Stand der Technik beschrieben. Dabei ist hervorzuheben, daß die genannten Rezepturen die Erfindung nicht begrenzen oder in irgendeiner Weise einschränken.

### Rezeptur 1:

| | | |
|---|---|---|
| | **Spirulina platensis** (Cyanophyceae), als (vom) lebende(n) Vegetationskörper gewonnen, getrocknet und pulverisiert, | 9800,00 g |
| und | **Kieselgur** (Diatomeae) | 100,00 g |
| und | **Curcuma longa** (Zingiberaceae) Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 2:

| | | |
|---|---|---|
| | **Spirulina platensis** (Cyanophyceae), als (vom) lebende(n) Vegetationskörper gewonnen, getrocknet und pulverisiert, | 9800,00 g |
| und | **Kieselgur** (Diatomeae) | 100,00 g |
| und | **Curcuma longa** (Zingiberaceae) wässriger Extrakt (Tumeric Extrakt), mit ca. 50 % Gehalt an Protein, getrocknet und pulverisiert | 100,00 g |

werden sorgfältig gemischt und anschließend zu Tabletten mit einem Gewicht von etwa 400 mg verpreßt.

### Rezeptur 3:

| | | |
|---|---|---|
| | **Spirulina platensis** (Cyanophyceae), als (vom) lebende(n) Vegetationskörper gewonnen, getrocknet und pulverisiert, | 9900,00 g |
| und | **Kieselgur** (Diatomeae) | 100,00 g |

werden sorgfältig gemischt und anschließend zu Tabletten mit einem Gewicht von etwa 400 mg verpreßt.

### Rezeptur 4:

| | | |
|---|---|---|
| | **Spirulina platensis** (Cyanophyceae), als (vom) lebende(n) Vegetationskörper gewonnen, getrocknet und pulverisiert, | 9900,00 g |
| und | **Aerosil** | 100,00 g |

werden sorgfältig gemischt und anschließend zu Tabletten mit einem Gewicht von etwa 400 mg verpreßt.

### Rezeptur 5:

| | | |
|---|---|---|
| | **Spirulina platensis** (Cyanophyceae), als (vom) lebende(n) Vegetationskörper gewonnen, getrocknet und pulverisiert, | 9900,00 g |
| und | **Lithotamnium calcareum** (Rhodophyceae) als fossiles Material gewonnen und pulverisiert | 100,00 g |

werden sorgfältig gemischt und anschließend zu Tabletten mit einem Gewicht von etwa 400 mg verpreßt.

### Rezeptur 6:

| | |
|---|---|
| **Spirulina platensis** (Cyanophyceae), als (vom) lebende(n) Vegetationskörper gewonnen, getrocknet und pulverisiert, | 10000,00 g |

werden sorgfältig gemischt und anschließend zu Tabletten mit einem Gewicht von etwa 400 mg verpreßt.

### Rezeptur 7:

| | | |
|---|---|---|
| | **Chlorella sp (Chlorophyceae)** aufgebrochene Zellen, getrocknet und pulverisiert, | 9800,00 g |
| und | **Kieselgur** (Diatomeae) | 100,00 g |
| und | **Curcuma longa** (Zingiberaceae) Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 8:

| | | |
|---|---|---|
| | **Aphanizomenon flos aqua** (Cyanophyceae) ("Klamath Alge") getrocknet und pulverisiert, | 9800,00 g |
| und | **Kieselgur** (Diatomeae) | 100,00 g |
| und | **Curcuma longa (Zingiberaceae)** Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 9:

| | | |
|---|---|---|
| | **Ascophyllum nodosum** (Phaeophyceae) getrocknet und pulverisiert, | 9800,00 g |
| und | **Kieselgur** (Diatomeae) | 100,00 g |
| und | **Curcuma longa (Zingiberaceae)** Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 10:

| | | |
|---|---|---|
| | **Hordeum vulgare** (Gramineae; Gerstengras) getrocknet und pulverisiert, | 9800,00 g |
| und | **Kieselgur** (Diatomeae) | 100,00 g |
| und | **Curcuma longa (Zingiberaceae)** Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 11:

| | | |
|---|---|---|
| | **Triticum turgidum** (Gramineae; Kamutgras) getrocknet und pulverisiert, | 9800,00 g |
| und | **Kieselgur** (Diatomeae) | 100,00 g |
| und | **Curcuma longa (Zingiberaceae)** Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 12:

| | | |
|---|---|---|
| | **Medicago sativa** (Fabaceae; Alfalfa) getrocknet und pulverisiert, | 9800,00 g |
| und | **Kieselgur** (Diatomeae) | 100,00 g |
| und | **Curcuma longa (Zingiberaceae)** Trockenextrakt, pulverisiert, Curcumingehalt ca. 95 Gew.% | 100,00 g |

werden sorgfältig gemischt und anschließend zu Tabletten mit einem Gewicht von etwa 400 mg verpreßt.

### Rezeptur 13:

| | | |
|---|---|---|
| | **Spirulina platensis** (Cyanophyceae), als (vom) lebende(n) Vegetationskörper gewonnen, getrocknet und pulverisiert, | 9400,00 g |
| und | **Kieselgur** (Diatomeae) | 100,00 g |
| und | **Vitamin C** vorzugsweise als Vitamin C-Calcium-Chelat | 500,00 g |

werden sorgfältig gemischt und anschließend zu Tabletten mit einem Gewicht von etwa 400 mg verpreßt.

### Rezeptur 14:

| | | |
|---|---|---|
| | **Spirulina platensis** (Cyanophyceae), als (vom) lebende(n) Vegetationskörper gewonnen, getrocknet und pulverisiert, | 7800,00 g |
| und | **Kieselgur** (Diatomeae) | 100,00 g |
| und | **Lithotamnium calcareum** (Rhodophyceae) als fossiles Material gewonnen und pulverisiert | 2000,00 g |
| und | **Curcuma longa** (Zingiberaceae) wässriger Extrakt (Tumeric Extrakt), mit ca. 50 % Gehalt an Protein, getrocknet und pulverisiert | 100,00 g |

werden sorgfältig gemischt und anschließend zu Tabletten mit einem Gewicht von etwa 400 mg verpreßt.

### Rezeptur 15:

| | | |
|---|---|---|
| | **Spirulina platensis** (Cyanophyceae), als (vom) lebende(n) Vegetationskörper gewonnen, getrocknet und pulverisiert, | 7900,00 g |
| und | **Lithotamnium calcareum** (Rhodophyceae) als fossiles Material gewonnen und pulverisiert | 2000,00 g |
| und | **Curcuma longa** (Zingiberaceae) wässriger Extrakt (Tumeric Extrakt), mit ca. 50 % Gehalt an Protein, getrocknet und pulverisiert | 100,00 g |

werden sorgfältig gemischt und anschließend zu Tabletten mit einem Gewicht von etwa 400 mg verpreßt.

### Rezeptur 16:

| Shnonend getrocknetes **Fructpulver #3** aus ganzen Früchten der Arten | |
|---|---|
| Apfel pectin / Faserstoffe | 35% |
| Citrus Pectin | 25% |
| Fruit blend 1071* | 20% |
| Apfel, Orange, Ananas (gleiche Teile) | 18% |
| Heidelbeere Noni Extract (5:1)(Morinda citrifolia) | 1% 1% |

| | |
|---|---|
| * (Moosbeere (cranbery), Apfel, Weintrauben, Grapefruit, Orange, Ananas, Zitrone, Limone, Kirsche, Wassermelone, Birne, Mango, Himbeere, Papaya, Tangerine, Aprikose) | |

| | | |
|---|---|---|
| | als (vom) lebende(n) Vegetationskörper gewonnen, getrocknet und pulverisiert, | 6150,00 g |
| und | **Inulin** (Zichorienextrakt) | 1000,00 g |
| und | **Kieselgur** (Diatomeae) | 100,00 g |
| und | **Lithotamnium calcareum** (Rhodophyceae) als fossiles Material gewonnen und pulverisiert | 2000,00 g |
| und | **Curcuma longa (Zingiberaceae)** | |
| Trockenextrakt, pulverisiert, Curcumingehalt ca. 95 Gew.% | | 750,00 g |

werden sorgfältig gemischt und anschließend zu Tabletten mit einem Gewicht von etwa 400 mg verpreßt.

| Shnonend getrocknetes **Fructpulver #3** aus ganzen Früchten der Arten | |
|---|---|
| Apfel pectin / Faserstoffe | 35% |
| Citrus Pectin | 25% |
| Fruit blend 1071* | 20% |
| Apfel, Orange, Ananas (gleiche Teile) | 18% |
| Heidelbeere Noni Extract (5:1) (Morinda citrifolia) | 1% |

| | |
|---|---|
| * (Moosbeere (cranbery), Apfel, Weintrauben, Grapefruit, Orange, Ananas, Zitrone, Limone, Kirsche, Wassermelone, Birne, Mango, Himbeere, Papaya, Tangerine, Aprikose) | |

| | | |
|---|---|---|
| | als (vom) lebende(n) Vegetationskörper gewonnen, getrocknet und pulverisiert, | 6900,00 g |
| und | **Inulin** (Zichorienextrakt) | 1000,00 g |
| und | **Kieselgur** (Diatomeae) | 100,00 g |
| und | **Lithotamnium calcareum** (Rhodophyceae) als fossiles Material gewonnen und pulverisiert | 2000,00 g |

werden sorgfältig gemischt und anschließend zu Tabletten mit einem Gewicht von etwa 400 mg verpreßt.

Die Tabletten der Rezeptur 16 weisen eine wesentlich höhere ganzheitliche Qualität nach der Biophotonen-Analyse auf als die Tabletten nach Rezeptur 17.

Anstelle von Curcuma longa konnen in den betreffenden Rezepturen erfindungsgemäß auch andere Curcuma-Arten verwendet werden.

Das in den Rezepturen verwendete Kieselgur weist vorzugsweise die nachfolgend genannten physikalischen Eigenschaften auf:

| Farbe: | weiß |
|---|---|
| Schüttdichte | 220 g/l |
| Wassergehalt | 1,0 % |
| pH-Wert | 9,0-10,0 |
| Naßdichte | 420g/l |
| Grobteile, nicht anschwemmbar | 0,1% |
| Weissgrad | 90-92 |
| Ölabsorption | 170% |
| Korngröbeße > 43 µm | maximal 2 % |

Ein derartiges Kieselgur ist beispielsweise bei United Minerals GmbH & Co KG, D 29633 Münster unter der technischen Bezeichnung Kieselgur MW 27, Produktnummer 4101 erhältlich.

Ein Trockenextrakt von Curcuma longa, wie er der Art nach in den Rezepturen (1,7 -12, 14 und 15) vorgesehen ist, und ein wässriges Extrakt von Curcuma longa, dem sogenannten Turmeric-Extrakt, wie in Rezeptur 2 vorgeschrieben, wird im Handel von Arjuna Natural Extracts Ltd., Cochin 683 101, Indien, unter der technischen Bezeichnung Curcumin Powder (für den Trocken-extrakt) bzw. Turmeric Extract angeboten.

Das in der Rezeptur 13 angegebene Vitamin C - Calcium-Chelat kann beispielsweise im Handel unter der technischen Bezeichnung Ester-C von Intercal Corp. bezogen werden.

Das in den Rezepturen (1, 7 - 12, 14 und 15) eingesetzte Trockenextrakt von Curcuma longa ist beispielsweise bei Arjuna Natural Extracts Ltd., Cochin 683 101, Indien, unter der technischen Bezeichnung Curcumin Powder erhältlich.

Sämtliche Tabletten haben eine bikonvexe Form mit einem Durchmesser von ca. 11 mm und einer Dicke bzw. Höhe von ca 3 mm und sind in einer Tablettenpresse der Art Fette P2200 mit einer Stempelform EU 19, einer Vorpreßkraft von ca. 70 kN, einer Preßkraft von ca. 85 kN und einer Fülltiefe von ca. 9 mm hergestellt.

Die Bestimmung der ganzheitlichen Qualität der erfindungsgemäßen Zubereitungen bzw. Tabletten kann mit Hilfe der Biophotonen-Analyse erfolgen. Die Biophotonen-Analyse ist eine "ganzheitliche" Analysenmethode. Die Bedeutung von "ganzheitlich" in diesem Zusammen-hang entspricht beispielsweise dem Zusammenspiel eines Musikorchesters während eines Konzertes, wobei die Qualität des Konzertes nur dadurch bewertet werden kann, daß das Zusammenspiel aller Instrumente geprüft und beurteilt wird.

Nachfolgend sind die Durchführung und Ergebnisse einer Biophotonen-Analyse an Tabletten, die nach den Rezepturen 1 bis 6 hergestellt wurden, wiedergegeben:

Es wurden Tabletten gemäß den vorstehend beispielhaft genannten Rezepturen 1 bis 6 hergestellt. Diese Tablettenproben "Rezp. 1, Rezp. 2, ..., Rezp. 6" wurden 2 Wochen bei 30° C im offenen Probenglas gelagert. Danach wurden die Tabletten der jeweiligen Probe pulverisiert. Von jeder Tablettenprobe wurde jeweils 4 g Pulver in je eine Quarzküvette gegeben und die Küvetten für 10 Minuten im Dunkeln gehalten (Dunkeladaption). Anschließend wurden die einzelnen Küvetten bzw. Proben in einem handelsüblichen Photometer den folgenden Untersuchungen unterworfen:

| | |
|---|---|
| (1) | Messung der Eigen(Licht-)-abstrahlung (= Eigenemission) der Probe = Bestimmung von "DA" Meßzeitintervall: 40 msec |
| (2) | Messung der Photonenemission nach spektraler Anregung mit Licht = Bestimmung von "NB 1" Meßzeitintervall: 40 msec Anregungsdauer: 10 sec Anregungsleistung: 75 Watt Anregungswellenlänge: Weißlicht |

Das Ergebnis dieser Untersuchungen ist in Tabelle 1, Spalten 1 und 2 (jeweils Mittelwert X und Standardabweichung) dargestellt.

Ergänzend zu den Photonenemissionsmessungen wurde

| | |
|---|---|
| (3) | die Abklingzeit, d.h. die Zeit gemessen = Bestimniung von "AH", |
| (4) | die Abweichung von der hyperbolischen Abklingfunktion nach Lichtanregung (= Kohärenzgrad [Ordnungsgrad] der Lichtspeicherung) gemessen = Bestimmung von "CHIH", |
| (5) | die Abweichung von der exponentiellen Abklingfunktion nach Lichtanregung (= Struktur des Lichtspeichersystems) gemessen = Bestimmung von "CHIE" |
| (6) | der "CHIEH"-Wert als weiteres relatives Maß für die Ordnung der Werte nach Anregung mit Licht gemessen. |

Die gemessenen Daten zu den Parametern DA, NB 1, AH, CHIE, CHIH und CHIEH werden zueinander in Relation gesetzt und eine Qualitätsrangfo 1 ge ermittelt, d.h. mit den betreffenden Daten wird eine sog. Faktorenanlayse durchgeführt. Die Ergebnisse dieser Faktorenanalyse sind in Tabelle 2 dargestellt.
Hinsichtlich näherer Angaben zur Durchführung der Messungen, zur Bestimmung der Werte für die Parameter AH, NB1, DA, CHIH, CHIE und CHIEH, und zur Ermittlung der in Tabelle 2 und Tabelle 3 aufgelisteten Faktor-Werten wird auf die genannte Literatur, insbesondere von F. A. Popp verwiesen.

Es folgen die Tabellen 1, 2 und 3.

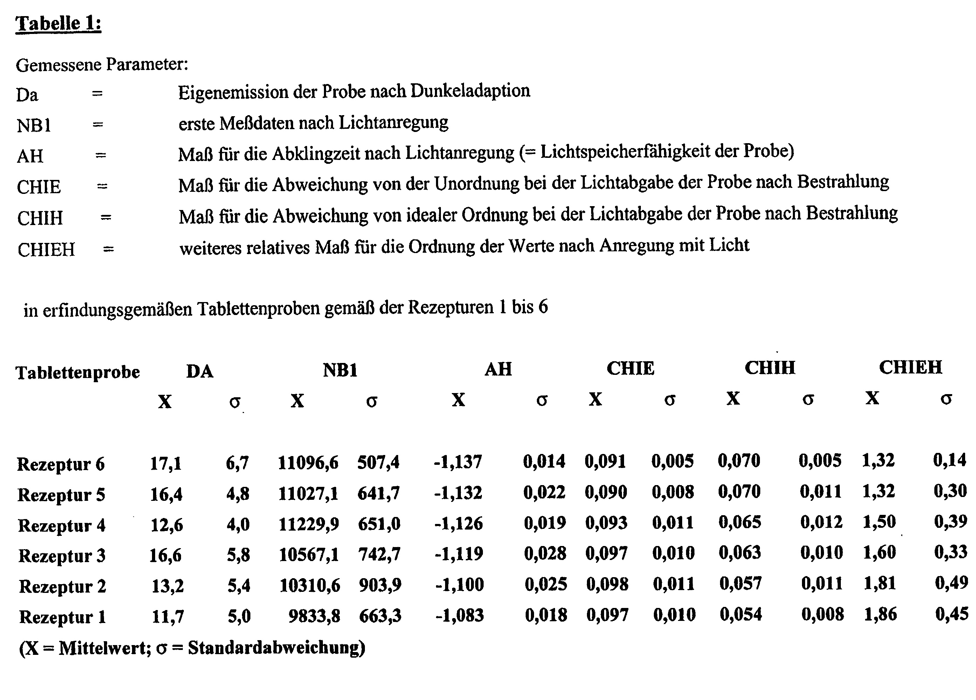

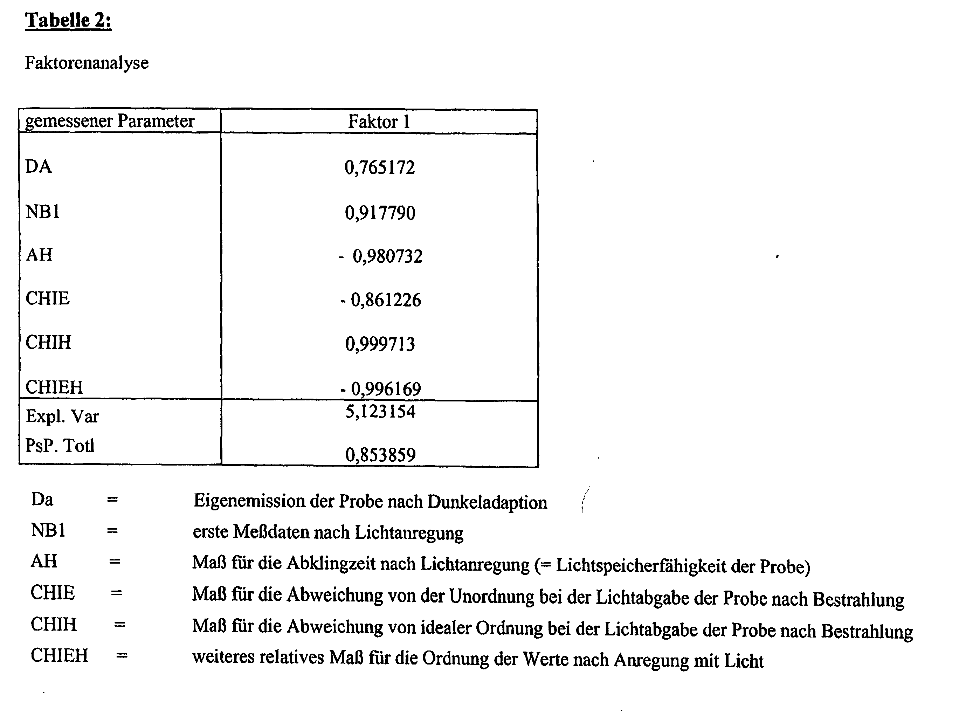

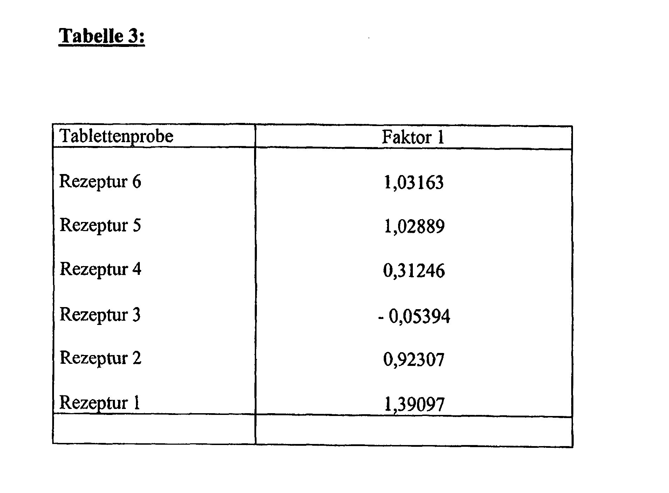

## Patentansprüche

1. Zubereitung für oral zu verabreichende Nahrungsergänzungsmittel in Form von Tabletten für Mensch und/oder Tier auf der Basis von pflanzlichem Material natürlichen Ursprungs, enthaltend Kalk-bildende Algen und/oder Kieselsäure bildende Algen und/oder Teile dieser Algen, **dadurch gekennzeichnet, dass** die Zubereitung ein natürliches Antioxidans, ausgewählt aus der Gruppe von Astragalus sp. (Leguminosae/Fabaceae), Piper nigrum (Piperaceae), Capsicum sp. (Solanaceae), Apium graveolens (Umbellifereae), Cacao theobroma (Sterculiaceae), Eleutherococcus senticosus (Araliaceae), Zingiber officinalis (Zingiberaceae), Pueraria lobata (Leguminosea/Fabaceae), Citrus limon oder Citrus paradisi (Rufaceae), Vitis vinifera (Vitaceae), Sylibum marianum, (Asteraceae/Compositae), Boswellia sp. (Burseraceae), Rosmarinus officinalis, (Labiatea/Laminaceae), Salvia officinalis (Labiatae/Laminaceae), Schisandra sp. (Schisandraceae) und/oder Camellia sinensis (Theaceac) und/oder Curcumin-reichen Trockenextrakten von Curcuma sp. (Zingiberaceae) und/oder wässrige Extrakte von Curcuma sp. (Tumeric-Extrakte mit hohem Proteingehalt) und/oder Vitamin C als Zusatz (Additiv) enthält.

2. Zubereitung nach Anspruch 1, **dadurch gekennzeichnet, dass** das pflanzliche Material ganz oder teilweise aus Blaualgen (Cyanophyceae) und/oder Grünalgen (Chlorophyceae) und/oder Braunalgen (Phaeophyceae) und/oder Teilen davon besteht.

3. Zubereitung nach Anspruch 2, **dadurch gekennzeichnet, dass** die Blaualgen aus der systematischen Gruppe Spirulina ausgewählt sind.

4. Zubereitung nach Anspruch 1, **dadurch gekennzeichnet, dass** das pflanzliche Material chlorophyllreiches Extrakt höherer Pflanzen enthält.

5. Zubereitung nach Anspruch 4, **dadurch gekennzeichnet, dass** die höheren Pflanzen im wesentlichen Gräser sind, insbesondere Weizen und/oder Gerste und/oder Kamut.

6. Zubereitung nach einem der Ansprüche 1 bis 5, **dadurch gekennzeichnet, dass** die Kalk bildenden Algen im wesentlichen Rotalgen (Rhodophyceae) sind.

7. Zubereitung nach Anspruch 6, **dadurch gekennzeichnet, dass** die Rotalgen aus der systematischen Gruppe Lithothamnium stammen, vorzugsweise der Art Lithothamnium calcareum sind.

8. Zubereitung nach einem der Ansprüche 1 bis 7, **dadurch gekennzeichnet, dass** die Kieselsäure bildenden Algen im wesentlichen Kieselalgen (Bacillariophyceae; Diatomeae) sind.

9. Zubereitung nach einem der Ansprüche 1 bis 8, **dadurch gekennzeichnet, dass** die Kieselsäure bildenden Algen als Kieselgur vorliegen.

10. Zubereitung nach einem der Ansprüche 1 bis 9, **dadurch gekennzeichnet, dass** die Kalk bildenden Algen und/oder Kieselsäure bildenden Algen und/oder Teile dieser Algen in einer Menge von 0,1 bis 50 Gewichts-%, vorzugsweise von 0,3 bis 35% und insbesondere von 10 bis 30%, jeweils bezogen auf das Gesamtgewicht der Zubereitung, vorliegen.

11. Zubereitung nach einem der Ansprüche 1 bis 9, **dadurch gekennzeichnet, dass** die Kalk bildenden Algen und/oder Kieselsäure bildenden Algen und/oder Teile dieser Algen in einer Menge von 0,05 bis 10 Gewichts-%, vorzugsweise von 0,1 bis 3% und insbesondere von 0,3 bis 1%, jeweils bezogen auf das Gesamtgewicht der Zubereitung, vorliegen.

12. Zubereitung nach einem der Ansprüche 1 bis 11, **dadurch gekennzeichnet, dass** das Antioxidians in Form eines Trockenextrakts und/oder eines wässrigen Extrakts von Curcuma sp. (Zingiberaceae) vorliegt.

13. Zubereitung nach einem der Ansprüche 1 bis 12, **dadurch gekennzeichnet, dass** das Antioxidians Vitamin C enthält.

14. Zubereitung nach einem der Ansprüche 1 bis 13, **dadurch gekennzeichnet, dass** sie zu wenigstens 40 Gewichtsprozent, vorzugsweise wenigstens 60 Gewichtsprozent und besonders bevorzugt wenigstens 70 Gewichtsprozent aus dem chlorophyllreichen, pflanzlichen Material besteht.

15. Zubereitung nach einem der Ansprüche 1 bis 14, **dadurch gekennzeichnet, dass** sie zu wenigstens 70 Gewichtsprozent, vorzugsweise wenigstens 80 Gewichtsprozent und besonders bevorzugt wenigstens 90 Gewichtsprozent aus dem chlorophyllreichen, pflanzlichen Material besteht.

16. Zubereitung nach einem der Ansprüche 1 bis 15, **dadurch gekennzeichnet, dass** sie einen Zusatz an Sprengmittel enthält, vorzugsweise Reis oder Kartoffelmehl.

17. Verwendung von einem oder mehreren natürlichen Antioxidantien, ausgewählt aus der Gruppe von Astragalus sp. (Leguminosae/Fabaceae), Piper nigrum (Piperaceae), Capsicum sp. (Solanaceae), Apium graveolens (Umbellifereae), Cacao theobroma (Sterculiaceae), Eleutherococcus senticosus (Araliaceae), Zingiber officinalis (Zingiberaceae), Pueraria lobata (Leguminosea/Fabaceae), Citrus limon oder Citrus paradisi (Rufaceae), Vitis vinifera (Vitaceae), Sylibum marianum, (Asteraceae/Compositae), Boswellia sp. (Burseraceae), Rosmarinus officinalis, (Labiatea/Laminaceae), Salvia officinalis (Labiatae/Laminaceae), Schisandra sp. (Schisandraceae) und/oder Camellia sinensis (Theaceac) und/oder Curcumin-reichen Trockenextrakten von Curcuma sp. (Zingiberaceae) und/oder wässrige Extrakte von Curcuma sp. (Tumeric-Extrakte mit hohem Proteingehalt) und/oder Vitamin C als Zusatz (Additiv) bei der Herstellung einer Zubereitung für oral zu verabreichende Nahrungsergänzungsmittel in Form von Tabletten für Mensch und/oder Tier auf der Basis von chlorophyllreichem, pflanzlichem Material natürlichen Ursprungs und unter Verwendung von Kalk bildenden Algen und/oder Kieselsäure bildenden Algen und/oder Teilen dieser Algen.

## Claims

1. Preparation for food supplements to be administered orally in the form of tablets for humans and/or animals, based on plant material of natural origin, comprising lime-forming algae and/or silicic acid-forming algae and/or parts of these algae, **characterized in that** the preparation contains a natural antioxidant selected from the group consisting of Astragalus sp. (Leguminosae/Fabaceae), Piper nigrum (Piperaceae), Capsicum sp. (Solanaceae), Apium graveolens (Umbellifereae), Theobroma cacao (Sterculiaceae), Eleutherococcus senticosus (Araliaceae), Zingiber officinalis (Zingiberaceae), Pueraria lobata (Leguminosae/Fabaceae), Citrus limon or Citrus paradisi (Rufaceae), Vitis vinifera (Vitaceae), Silybum marianum (Asteraceae/Compositae), Boswellia sp. (Burseraceae), Rosmarinus officinalis (Labiatae/Laminaceae), Salvia officinalis (Labiatae/Laminaceae), Schisandra sp. (Schisandraceae) and/or Camellia sinensis (Theaceae) and/or curcumin-rich dry extracts of Curcuma sp. (Zingiberaceae) and/or aqueous extracts of Curcuma sp. (turmeric extracts having a high protein content) and/or vitamin C as an additive.

2. Preparation according to Claim 1, **characterized in that** the plant material consists completely or partially of blue algae (Cyanophyceae) and/or green algae (Chlorophyceae) and/or brown algae (Phaeophyceae) and/or parts thereof.

3. Preparation according to Claim 2, **characterized in that** the blue algae are selected from the systematic group Spirulina.

4. Preparation according to Claim 1, **characterized in that** the plant material contains a chlorophyll-rich extract of higher plants.

5. Preparation according to Claim 4, **characterized in that** the higher plants are mainly grasses, in particular wheat and/or barley and/or kamut.

6. Preparation according to one of Claims 1 to 5, **characterized in that** the lime-forming algae are mainly red algae (Rhodophyceae).

7. Preparation according to Claim 6, **characterized in that** the red algae are derived from the systematic group Lithothamnium, preferably of the species Lithothamnium calcareum.

8. Preparation according to one of Claims 1 to 7, **characterized in that** the silicic acid-forming algae are mainly diatoms (Bacillariophyceae; Diatomeae).

9. Preparation according to one of Claims 1 to 8, **characterized in that** the silicic acid-forming algae are present as kieselguhr.

10. Preparation according to one of Claims 1 to 9, **characterized in that** the lime-forming algae and/or silicic acid-forming algae and/or parts of these algae are present in an amount of from 0.1 to 50% by weight, preferably from 0.3 to 35% and in particular from 10 to 30%, in each case based on the total weight of the preparation.

11. Preparation according to one of Claims 1 to 9, **characterized in that** the lime-forming algae and/or silicic acid-forming algae and/or parts of these algae are present in an amount of from 0.05 to 10% by weight, preferably from 0.1 to 3% and in particular from 0.3 to 1%, in each case based on the total weight of the preparation.

12. Preparation according to one of Claims 1 to 11, **characterized in that** the antioxidant is present in the form of a dry extract and/or of an aqueous extract of Curcuma sp- (Zingiberaceae).

13. Preparation according to one of Claims 1 to 12, **characterized in that** the antioxidant contains vitamin C.

14. Preparation according to one of Claims 1 to 13, **characterized in that** it consists to at least 40 per cent by weight, preferably at least 60 per cent by weight and particularly preferably at least 70 per cent by weight, of the chlorophyll-rich, plant material.

15. Preparation according to one of Claims 1 to 14, **characterized in that** it consists to at least 70 per cent by weight, preferably at least 80 per cent by weight and particularly preferably at least 90 per cent by weight, of the chlorophyll-rich, plant material.

16. Preparation according to one of Claims 1 to 15, **characterized in that** it contains an addition of disintegrant, preferably rice or potato flour.

17. Use of one or more natural antioxidants, selected from the group consisting of Astragalus sp. (Leguminosae/Fabaceae), Piper nigrum (Piperaceae), Capsicum sp. (Solanaceae), Apium graveolens (Umbellifereae), Theobroma cacao (Sterculiaceae), Eleutherococcus senticosus (Araliaceae), Zingiber officinalis (Zingiberaceae), Pueraria lobata (Leguminosae/Fabaceae), Citrus limon or Citrus paradisi (Rufaceae), Vitis vinifera (Vitaceae), Silybum marianum (Asteraceae/Compositae), Boswellia sp. (Burseraceae), Rosmarinus officinalis (Labiatae/Laminaceae), Salvia officinalis (Labiatae/Laminaceae), (Schisandra sp. Schisandraceae) and/or Camellia sinensis Theaceae) and/or curcumin-rich dry extracts of Curcuma sp. (Zingiberaceae) and/or aqueous extracts of Curcuma sp. (turmeric extracts having a high protein content) and/or vitamin C as an additive in the production of a preparation for food supplements to be administered orally in the form of tablets for humans and/or animals, based on chlorophyll-rich, plant material of natural origin and using lime-forming algae and/or silicic acid-forming algae and/or parts of these algae.

## Revendications

1. Composition pour un complément alimentaire à administrer par voie orale sous forme de comprimés pour l'homme et/ou l'animal à base d'une matière végétale d'origine naturelle, contenant des algues formant du calcaire et/ou des algues formant de la silice ou des parties de ces algues, **caractérisée en ce que** la composition contient comme additif un antioxydant naturel, choisi dans le groupe constitué par Astragalus sp. (Leguminosae/Fabaceae), Piper nigrum (Piperaceae), Capsicum sp. (Solanaceae), Aplum graveolens (Umbellifereae), Cacao theobroma (Stercullaceae), Eleutherococcus senticosus (Araliaceae), Zingiber officinalis (Zingiberaceae), Pueraria lobata (Leguminosea/Fabaceae), Citrus limon ou Citrus paradisi (Rufaceae), Vitis vinifera (Vitaceae),Sylibum marianum,(Asteraceae/Compositae), Boswellia sp. (Burseraceae), Rosmarinus officinalis, (Labiatea/Laminaceae),Salvia officinalis (Labiatae /Laminaceae),Schisandra sp.(Schisandraceae) et/ou Camellia sinensis (Theaceac) et/ou des extraits secs riches en curcumine provenant de Curcuma sp. (Zingiberaceae) et/ou des extraits aqueux de Curcuma sp. (extraits tumériques présentant une teneur élevée en protéines) et/ou de la vitamine C.

2. Composition selon la revendication 1, **caractérisée en ce que** la matière végétale est constituée totalement ou partiellement d'algues bleues (Cyanophyceae) et/ou d'algues vertes (Chlorophyceae) et/ou d'algues brunes (Phaeophyceae) et/ou de parties de celles-ci.

3. Composition selon la revendication 2, **caractérisée en ce que** les algues bleues sont choisies dans le groupe systématique des spirulines.

4. Composition selon la revendication 1, **caractérisée en ce que** la matière végétale contient un extrait riche en chlorophylle de plantes élevées.

5. Composition selon la revendication 4, **caractérisée en ce que** les plantes élevées sont essentiellement des graminées, en particulier le blé et/ou l'orge et/ou le kamut.

6. Composition selon l'une quelconque des revendications 1 à 5, **caractérisée en ce que** les algues formant du calcaire sont essentiellement des algues rouges (Rhodophyceae).

7. Composition selon la revendication 6, **caractérisée en ce que** les algues rouges proviennent du groupe systématique Lithothamnium, et sont de préférence du type Lithothamnium calcareum.

8. Composition selon l'une quelconque des revendications 1 à 7, **caractérisée en ce que** les algues formant de la silice sont essentiellement des algues siliceuses (Bacillariophyceae; Diatomeae).

9. Composition selon l'une quelconque des revendications 1 à 8, **caractérisée en ce que** les algues formant de la silice se trouvent sous forme de terres de diatomées.

10. Composition selon l'une quelconque des revendications 1 à 9, **caractérisée en ce que** les algues formant du calcaire et/ou les algues formant de la silice et/ou des parties de ces algues se trouvent en une quantité de 0,1 à 50% en poids, de préférence de 0,3 à 35% et en particulier de 10 à 30%, à chaque fois par rapport au poids total de la composition.

11. Composition selon l'une quelconque des revendications 1 à 9, **caractérisée en ce que** les algues formant du calcaire et/ou les algues formant de la silice et/ou des parties de ces algues se trouvent en une quantité de 0,05 à 10% en poids, de préférence de 0,1 à 3% et en particulier de 0,3 à 1%, à chaque fois par rapport au poids total de la composition.

12. Composition selon l'une quelconque des revendications 1 à 11, **caractérisée en ce que** l'antioxydant se trouve sous forme d'un extrait sec et/ou d'un extrait aqueux de Curcuma sp. (Zingiberaceae).

13. Composition selon l'une quelconque des revendications 1 à 12, **caractérisée en ce que** l'antioxydant contient de la vitamine C.

14. Composition selon l'une quelconque des revendications 1 à 13, **caractérisée en ce qu'**elle est constituée à raison d'au moins 40% en poids, de préférence d'au moins 60% en poids et de manière particulièrement préférée d'au moins 70% en poids de matière végétale, riche en chlorophylle.

15. Composition selon l'une quelconque des revendications 1 à 14, **caractérisée en ce qu'**elle est constituée à raison d'au moins 70% en poids, de préférence d'au moins 80% en poids et de manière particulièrement préférée d'au moins 90% en poids de matière végétale, riche en chlorophylle.

16. Composition selon l'une quelconque des revendications 1 à 15, **caractérisée en ce qu'**elle contient un ajout d'agent de désintégration, de préférence du riz ou de la farine de pommes de terre.

17. Utilisation d'un ou de plusieurs antioxydants naturels, choisis dans le groupe constitué par Astragalus sp. (Leguminosae/Fabaceae), Piper nigrum (Piperaceae), Capsicum sp. (Solanaceae), Aplum graveolens (Umbellifereae), Cacao theobroma (Stercullaceae), Eleutherococcus senticosus (Araliaceae), Zingiber officinalis (Zingiberaceae), Pueraria lobata (Leguminosea/Fabaceae), Citrus limon ou Citrus paradisi (Rufaceae), Vitis vinifera (Vitaceae),Sylibum marianum,(Asteraceae/ Compositae) ,Boswellia sp.(Burseraceae),Rosmarinus officinalis, (Labiatea/Laminaceae),Salvia officinalis (Labiatae/Laminaceae),Schisandra sp. (Schisandraceae) et/ou Camellia sinensis (Theaceac) et/ou les extraits secs riches en curcumine de Curcurma sp. (Zingiberaceae) et/ou les extraits aqueux de Curcuma sp. (extraits tumériques avec une teneur élevée en protéines) et/ou de la vitamine C comme additif lors de la préparation d'une composition pour des compléments alimentaires à administrer par voie orale sous forme de comprimés pour l'homme et/ou l'animal à base de matière végétale, riche en chlorophylle, d'origine naturelle et avec utilisation d'algues formant du calcaire et/ou d'algues formant de la silice et/ou de parties de ces algues.
